# EUROPEAN PATENT SPECIFICATION

(11) **EP 2 061 744 B1**
(45) Date of publication and mention of the grant of the patent: **30.07.2014**
(21) Application number: 07801127.7
(22) Date of filing: 06.09.2007
(51) Int. Cl.: C07C 51/02, C07C 51/09, C07C 51/43, B01D 61/42

(54) **PROCESS FOR RECOVERING TEREPHTHALIC ACID**
VERFAHREN ZUR RÜCKGEWINNUNG VON TEREPHTHALSÄURE
PROCEDE DE RECUPERATION DE L'ACIDE TEREPHTALIQUE

(30) Priority: 08.09.2006 CZ 20060550
(43) Date of publication of application: 27.05.2009
(73) Proprietor: Ustav Chemickych Procesu Akademie Ved Ceské Republiky, 16500 Prague 6 (CZ)
(72) Inventor: VESELY, Vaclav, 150 00 Prague 5 (CZ); DRAHOS, Jiri, 16500 Prague 6 (CZ); SIREK, Milan, 12000 Prague 2 (CZ)
(74) Representative: Rezac, Petr
(86) International application number: PCT/CZ2007/000086
(87) International publication number: WO 2008/028429

(56) References cited:
- US-A- 4 092 230
- US-A- 4 093 528
- US-B1- 6 312 582

## Description

### Field of invention

The invention relates to the process for recovering terephthalic acid from its salts solution, to produce pure, crystalline terephthalic acid.

### Description of prior art

Terephthalic acid is almost insoluble in water at a room temperature. This property is utilized in its isolation or purification respectively. Nevertheless, terephthalic acid may form three significantly soluble salts, namely ammonium, sodium and potassium salts. In praxis, terephthalic acid is recovered from solutions of said salts through precipitation by an acid typically a mineral acid. During this reaction an insoluble terephthalic acid precipitates and the corresponding mineral acid salts are formed. The history of such reaction may be demonstrated by way of the following example:

disodium terephthalic acid salt + H₂SO₄ → terephthalic acid + Na₂SO₄

Terephthalic acid is than recovered from the reaction mixture by precipitation and filtration.

U.S. pat No. 3,544,622 discloses an alkaline glycolysis of polyethylene terephthalate, hereinafter also referred to as "PET", at a raised temperature and under atmospheric pressure. The reaction mixture is filtered and sodium salt of terephthalic acid is obtained. This sodium salt is washed and impurities are then separated by dissolution and filtration. Solution of sodium salt is then acidified by a mineral acid and terephthalic acid is recovered by filtration.

A method of base glycolysis of PET carried out by sodium or potassium hydroxide under atmospheric pressure and at a temperature of 140 to 180°C is disclosed in EP No. 587 751. The resulting terephthalic acid salt is dissolved in water. Impurities and glycol are extracted by alcohol and terephthalic acid is then obtained by precipitation of the extract by a mineral acid whereupon the precipitant is filtered and dried.

Another European patent No. 597 751 describes a method of decomposition of polyethylene terephthalate by caustic soda in a melt in an extruder. Again, the salt formed is separated from residuals by its dissolving in water and terephthalic acid is then isolated by neutralization by use of a mineral acid.

According to U.S. pat No. 5,395,858, polyethylene terephthalate is supplied to a boiling caustic soda solution. Ethylene glycol is volatilized. The residual sodium salt is then dissolved in water, acidified and the precipitated terephthalic acid is filtered off and dried.

U.S. pat No. 6,031,128 discloses also an alkaline hydrolysis of crushed polyethylene terephthalate. Terephthalic acid is isolated by application of a mineral acid and resulting filtrate containing a mineral acid salt (NaCl) is subjected to electro dialysis to obtain HCl. This acid is further reused to decompose terephthalic acid salt. The resulting mother liquor is returned to the hydrolyzing process.

According to the published Czech application for invention PV 2004-748 a two-stage hydrolysis is carried out whereby in the first stage alkaline hydrolysis and glycolysis is accomplished in an extruder and the resulting oligomeric products react in an aqueous solution of hydroxide of an alkali metal in the second stage.

However, the described methods of recovering terephthalic acid are accompanied by principal deficiency characterized by high consumption of chemicals and complicated processing of wastewater with high concentration of salts.

US 4,092,230 discloses_a process of electrolyzing an aqueous solution of potassium acid terephthalate introduced into the anode department of an electrolytic cell preferably in the presence of a potassium salt of an acid stronger than terephthalic acid, or monopotassium terephthalate, in the presence of an acid stronger than terephthalic acid to precipitate terephthalic acid and separating the terephthalic acid product. Monopotassium terephthalate may be obtained by conversion of terephthalonitrile to terephthalic acid by hydrolyzing terephthalonitrile in an aqueous medium containing selected potassium compounds.

In US 4,093,528 a process_for the preparation of terephthalic acid is described, which comprises electrolyzing disodium terephthalate introduced into the anode department of an electrolytic cell whereby terephthalic acid product is precipitated and subsequently separated. In a preferred embodiment the disodium terephthalate is obtained by aqueous hydrolysis of terephthalonitrile in the presence of a stoichiometric excess of sodium hydroxide.

US 6,312,582 shows a method_for recuperating from saponification products of alkaline polyterephthalate with soda where both terephthalate ions are in the acid form and the sodium ions are in the form of soda. In this method, the sodium terephthalate solution resulting from dissolving the saponification products are subjected to a step of electrochemical pre-acidification to bring the pH to 4 to 7. Thereafter an electrochemical acidification step by electrolysis is undertaken to precipitate the terephthalic acid in the anode section and recuperate the soda in the cathode section which can be recycled.

The reaction scheme presented in the above cited US patents disclose a method and device for recovery of terephthalic acid /TPA/ H₂TP/ in the process by which an alkali metal TPA salt is introduced into the anode compartment to induce the precipitation of TPA at the anode and transfer of alkaline metal ions through a cationic membrane into the cathode compartment to form alcaline hydroxide therein. Nevertheless, the terephthalic acid salt solutions contain impurities or admixtures and in the process of precipitation, terephthalic acid has a tendency to absorb on the surface of the growing crystals most of impurities present in the anode compartment and consequently, it may be very difficult to remove them economically by conventional methods, for example by applying a sorbent to obtain a pure acid."

The primary object of the present invention is to increase the yield of the respective processes and to optimize energy consumption so that the whole recycling technology including recovery of terephthalic acid as a significant part of the process would be economically acceptable with respect to actual prices of raw materials and energies.

### Summary of the invention

The object of the present invention is achieved and the described deficiencies overcome by providing a process for recovering terephthalic acid from its salts solution wherein the aqueous solution of terephthalic acid and electrolyte is prepared by chemical recycling of waste polyethylene terephthalate including an initial stage of alkaline hydroglycolysis, in which the waste polyethylene terephthalate is subjected in a reactor to alkaline hydroglycolysis by caustic soda or potassium hydroxide solution with simultaneous distilling off water from the reactor, the resulting suspension of terephthalic acid sodium salt crystals and glycol is then separated and diluted by water and after removing residual impurities by a sorbent the aqueous solution of a terephthalic acid salt is fed into a cathode compartment of an electro dialysis cell and an electrolyte into an anode compartment, the resulting salt and electrolyte solution is then subjected to electrolysis and terephthalic acid resulting from the reaction of terephthalic acid anions with the electrolyte cations in the anode compartment is withdrawn from the anode compartment and separated from the electrolyte by filtration.

The essential features of the invention and their advantages are further demonstrated and/or presented in more detail on various possible embodiments of the invention.

The terephthalic acid salt is selected from the group comprising ammonium, sodium or potassium salt.

The electrolyte is selected from the group comprising water, mineral acid or a salt thereof.

The concentration of aqueous solution of terephthalic acid is 2 - 15% by weight.

The electrolyte is sulfuric or nitric acid with a concentration from 0.01 M to 0.5 M.

The aqueous solution of terephthalic acid contains in addition a salt of a mineral acid with a concentration from 0.01 to 0.5 % by weight.

The terephthalic acid is withdrawn from the anode compartment when the electro dialysis current drops to a value equal to 15 to 20 % of its initial value.

The weight ratio of glycol to polyethylene terephthalate is from 1:1 to 1:10, preferably from 1:4 to 1:6.

The aqueous solution of hydroxide and glycol resulting from the process of electrolysis is returned to the initial stage of hydroglycolysis.

The invention relies on the fact that terephthalic acid is practically insoluble in water at a room temperature so that in the process of electrolytic decomposition of a salt thereof its anion is recombined with a hydrogen cation even in an aqueous solution so that the acid is precipitated in the solution in a form of a solid crystal phase. To speed up the process a diluted mineral acid solution or salts thereof or other types of available electrolytes may be used.

When utilizing the process according to the invention in recycling a waste polyethylene terephthalate a crushed PET is fed to an aqueous solution of ethylene glycol and alkali metal liquor. The suspension so obtained is mixed and boiled as long as water is evaporated. Almost insoluble terephthalic acid salt then precipitates from the reaction mixture. The reaction is completed by distilling off the residual water at controlled temperature. The salt is then separated from the redundant glycol and dissolved in water. From the resulting solution, residual coloring substances and undissolved components are removed by adsorption on the surface of activated charcoal. The colorless solution is supplied to an electrolyzer where ions are separated into cations and anions by action of electric current. The hydrogen ion obtained by water electrolysis reacts in the anode compartment with the terephthalic acid anion and terephthalic acid is precipitated from the solution in the crystalline form. The cathode liquor contains an aqueous solution of glycol and alkali metal hydroxide. This solution is returned into the first stage, where the PET glycolysis takes place. The anode liquor includes a suspension of terephthalic acid crystals that are separated by filtration, washed and dried. The resulting crystals represent one of the products of terephthalic acid recycling. The second product is ethylene glycol, which is obtained by distilling off the glycol resulting from the base glycolysis of PET after separation of terephthalic acid salts.

The process for recovering terephthalic acid according to the invention may be implemented on a simple electro dialysis device or an electrolysis device, in which the cathode compartment and the anode compartment are separated by a membrane that is currently available to serve such purposes. A substantial advantage is that the quantity of chemicals, which are necessary for carrying out the process is disproportionately lower than with a conventional process of precipitating by mineral acid. This results in overall reduction of production costs and elimination of any adverse effects on environment.

Another advantage of the invention is a high product yield and the possibility to optimize energy consumption so that the new recycling technology combination based on electro dialysis is economically acceptable as raw materials and energy costs are concerned. Moreover, in this recycling process the product of electrolysis originating in cathode liquor is returned back into the first stage, where the PET glycolysis takes place. "

### Detailed description of the preferred embodiments

### Example 1

An electro dialysis cell includes a cathode and an anode compartment separated from each other by an anion permeable membrane. This anode membrane was prepared by embedding an anion exchanger into a polyethylene material and in order to improve the membrane mechanical qualities the polyester or polyamide fabric was built in into the membrane. A sample of aqueous solution of sodium terephthalate of a concentration of 7 % by weight is charged into the cathode department and 0.1 M of sulfuric acid solution is added to the anode compartment. Upon connecting platinum electrodes to a source of 4 V direct current, hydrogen is generated at the cathode and oxygen at the anode. Simultaneously, terephthalic acid precipitates in the anode compartment and deposits on the bottom of the cell. When the direct current drops to 20 % of its original value, the sulfuric acid solution is withdrawn and the crystalline terephthalic acid is separated by filtration.

### Example 2

An aqueous solution of sodium terephthalate and sodium sulfate is charged into the cathode compartment of the same cell as described in Example 1. The concentration of terephthalate is 10 % by weight and the concentration of sulfate 0.2 % by weight. In the anode compartment, 0.05 M of sodium sulfate solution is added. Upon connecting platinum electrodes to a source of 4 V direct current, hydrogen is generated at the cathode and oxygen at the anode. Simultaneously, terephthalic acid is precipitated in the anode compartment and deposits on the bottom of the cell. Once the direct current drops to 20 % of its original value, the suspension is withdrawn and the crystalline terephthalic acid is separated by filtration.

### Example 3

An aqueous solution of ammonium terephthalate is charged into the same cell as described in Example 1. The concentration of terephthalate is 5 % by weight and the concentration of free ammonia is 0.2 % by weight. In the anode compartment, 0.05 M ammonium nitrate solution is added. Upon connecting the platinum electrodes to a source of 4 V direct current, ammonium is oxidized at the cathode and converted into nitric acid and the nitride ion is transported to the anode compartment by the action of direct current, where terephthalic acid is precipitated. Terephthalic acid deposits on the cell bottom and, when direct current drops to 15 % of its original value, the suspension is withdrawn and the crystalline terephthalic acid is separated by filtration.

### Example 4

In a three-liter sulfonation flask provided with a stirrer and distillation head, 1830 ml of recycled alkaline aqueous solution of ethylene glycol, 200 gr of crushed PET and 60 ml of pure glycol were inserted. The concentration of sodium hydroxide in the solution was 5 % by weight and the concentration of ethylene glycol was 15 % by weight. The mixture was heated at the rate given in the table:

| Time | 10:30 | 10:45 | 10:55 | 11:00 | 11:15 | 12:00 |
|---|---|---|---|---|---|---|
| Temperature °C | 30 start | 100 | 135 | 145 | 165 | 195 end |

Through the distillation head 1450 ml water were distilled off. After completion of the distillation at 195 °C, the heating was interrupted and the content of the flask was allowed to cool. The resulting 680 ml of mother liquor was decanted from the layer of deposited crystals. The suspension of crystals and glycol was diluted by the distillate from the reactor and a solution of sodium salt of terephthalic acid and glycol was obtained. This solution was heated in a flow-through pressure reactor to a temperature of 200 °C at atmospheric pressure. The retention time at this temperature was 15 min. After being hydrolyzed, the solution was cooled and activated powdered charcoal was added until the solution was decolorized. Thereafter the solution was filtered and fed into the cathode compartment of an electrolyzer equipped with Pt electrodes. 1 % sulfuric acid solution was fed into the anode compartment. The current density was 25 A/dm² and the voltage at electrodes 6 V. The resulting crystals of terephthalic acid were filtered off and washed in demineralized water. The glycol mother liquor was subjected to distillation under vacuum conditions (20 torr) at a temperature of 150 °C and the resulting glycol distilled off was returned to the process of the base glycolysis. Any crystals of terephthalic acid sodium salt precipitating from the distillation residue could be filtered off and put back into the dissolving stage before the hydrolyzing step in the pressure reactor.

### Industrial applicability

The invention may be used in a process of chemical recycling of polyethylene terephthalate (PET) products to recover terephthalate acid as a major component of this material. Said products include polyethylene terephthalate packings such as PET bottles and foils including film stripes. An intermediate of the recycling process is as a rule potassium or ammonium salt of terephthalic acid which may be converted into crystalline terephthalic acid according to the invention.

## Claims

1. A process for recovering terephthalic acid from its salts solution **characterized in that** the aqueous solution of terephthalic acid and electrolyte is prepared by chemical recycling of waste polyethylene terephthalate including an initial stage of alkaline hydroglycolysis, in which the waste polyethylene terephthalate is subjected in a reactor to alkaline hydroglycolysis by caustic soda or potassium hydroxide solution with simultaneous distilling off water from the reactor, the resulting suspension of terephthalic acid sodium salt crystals and glycol is then separated and diluted by water and after removing residual impurities by a sorbent the aqueous solution of a terephthalic acid salt is fed into a cathode compartment of an electro dialysis cell and an electrolyte into an anode compartment, the resulting salt and electrolyte solution is then subjected to electrolysis and terephthalic acid resulting from the reaction of terephthalic acid anions with the electrolyte cations in the anode compartment is withdrawn from the anode compartment and separated from the electrolyte by filtration.

2. The process for recovering terephthalic acid from its salts solution according to claim 1 **characterized in that** the terephthalic acid salt is selected from the group comprising ammonium, sodium or potassium salt.

3. The process for recovering terephthalic acid from its salts solution according to claim 1 and 2 **characterized in that** the electrolyte is selected from the group comprising water, mineral acid or a salt thereof.

4. The process for recovering terephthalic acid from its salts solution according to any of claims 1 to 3 **characterized in that** the concentration of the aqueous solution of terephthalic acid is 2 - 15% by weight.

5. The process for recovering terephthalic acid from its salts solution according to any of claims 1 to 4 **characterized in that** an electrolyte is sulfuric or nitric acid of a concentration from 0.01 M to 0.5 M.

6. The process for recovering terephthalic acid from its salts solution according to any of claims 1, 2 or 4 **characterized in that** the aqueous solution of terephthalic acid contains in addition a salt of a mineral acid with a concentration from 0.01 to 0.5 % by weight.

7. The process for recovering terephthalic acid from its salts solution according to any of claims 1 to 6 **characterized in that** terephthalic acid is withdrawn from the anode compartment when the electro dialysis current drops to a value from 15 to 20 % of its initial value.

8. The process for recovering terephthalic acid from its salts solution according to claim 1 **characterized in that** in the process of alkaline hydroglycolysis the weight ratio of glycol to polyethylene terephthalate is from 1:1 to 1:10, preferably from 1:4 to 1:6.

9. The process for recovering terephthalic acid from its salts solution according to claim 1 and 9 **characterized in that** the aqueous solution of hydroxide and glycol resulting from the process of electrolysis is returned to the initial stage of hydroglycolysis.

## Patentansprüche

1. Verfahren zur Rückgewinnung von Terephthalsäure aus Lösung deren Salze, **dadurch gekennzeichnet, dass** die wässrige Lösung der Terephthalsäure und Elektrolyt durch chemische Wiederaufbereitung des verbrauchten Polyethylenterephthalat, die als Anfangsstufe die alkalische Hydroglykolyse enthaltet, gefertigt wird, wobei das verbrauchte Polyethylenterephthalat in einem Reaktor der alkalischen Hydroglykolyse durch Natronlauge oder Kalilauge, unter gleichzeitigem Abdestillieren von Wasser aus dem Reaktor unterworfen wird, die resultierende Suspension von Terephthalsäure, Natriumsalz-Kristalle und Glykol dann abgetrennt und durch Wasser verdünnt wird, und nach Entfernen residualer Verunreinigungen durch ein Sorbentmittel, die wässrige Lösung des Salzes der Terephthalsäure in den Kathodenraum einer Elektrodialyse-Zelle und Elektrolyt in den Anodenraum zugeführt wird, das erhaltene Salz und die Elektrolytlösung anschließend einer Elektrolyse unterworfen werden und Terephthalsäure aus der Reaktion der Terephthalsäure Anionen mit den Elektrolyt Kationen im Anodenraum von dem Anodenraum entnommen und vom Elektrolyt durch das Filtrieren abgetrennt wird.

2. Verfahren zur Rückgewinnung von Terephthalsäure aus Lösung deren Salze nach Anspruch 1, **dadurch gekennzeichnet, dass** das Terephthalsäuresalz aus der Gruppe bestehend aus Ammonium-, Natrium- oder Kaliumsalz gewählt ist.

3. Verfahren zur Rückgewinnung von Terephthalsäure aus Lösung deren Salze nach Anspruch 1 und 2, **dadurch gekennzeichnet, dass** der Elektrolyt aus der Gruppe bestehend aus Wasser, Mineralsäure oder ein Salz davon gewählt ist.

4. Verfahren zur Rückgewinnung von Terephthalsäure aus Lösung deren Salze nach einem der Ansprüche 1 bis 3, **dadurch gekennzeichnet, dass** die Konzentration der wässrigen Lösung der Terephthalsäure 2 - 15 Gew% beträgt.

5. Verfahren zur Rückgewinnung von Terephthalsäure aus Lösung deren Salze nach einem der Ansprüche 1 bis 4, **dadurch gekennzeichnet, dass** der Elektrolyt Schwefelsäure oder Salpetersäure mit einer Konzentration von 0,01 M bis 0,5 M ist.

6. Verfahren zur Rückgewinnung von Terephthalsäure aus Lösung deren Salze nach einem der Ansprüche 1, 2 oder 4, **dadurch gekennzeichnet, dass** die wässrige Lösung der Terephthalsäure zusätzlich ein Salz einer Mineralsäure mit einer Konzentration von 0,01 bis 0,5 Gew% enthält.

7. Verfahren zur Rückgewinnung von Terephthalsäure aus Lösung deren Salze nach einem der Ansprüche 1 bis 6, **dadurch gekennzeichnet, dass** die Terephthalsäure aus dem Anodenraum abgezogen wird, wenn der Elektrodialysestrom auf einen Wert von 15 auf 20% des Ausgangswertes fällt.

8. Verfahren zur Rückgewinnung von Terephthalsäure aus Lösung deren Salze nach Anspruch 1 **dadurch gekennzeichnet, dass** das Gewichtsverhältnis von Glykol zu Polyethylenterephthalat während des Prozesses der Hydroglykolyse von 1: 1 bis 1: 10, vorzugsweise von 1: 4 bis 1: 6 ist.

9. Verfahren zur Rückgewinnung von Terephthalsäure aus Lösung deren Salze nach Anspruch 1 und 8, **dadurch gekennzeichnet, dass** die aus dem Elektrolyseverfahren erhaltene wässrige Lösung von Natriumhydroxid und Glykol zu der Anfangsstufe der Hydroglykolyse zurückgegeben wird.

## Revendications

1. Un procédé de récupération d'acide téréphtalique à partir de sa solution de sels, **caractérisé en ce que** la solution aqueuse de l'acide téréphtalique et d'électrolyte est préparée par le recyclage chimique des déchets de polyéthylène téréphtalate, ce procédé comprenant une étape initiale d'hydroglycolyse alcaline, dans laquelle le téréphtalate provenant des déchets de polyéthylène est soumise, dans un réacteur, à l'hydroglycolyse par la soude caustique ou une solution d'hydroxyde de potassium avec élimination simultanée de l'eau par distillation à partir du réacteur, la suspension résultante d'acide téréphtalique, den cristaux de sel de sodium et de glycol est alors séparée et diluée dans l'eau et après élimination des impuretés résiduelles présentes dans la suspension par un adsorbant, la solution aqueuse de sel d'acide téréphtalique est versée dans un compartiment d'cathode d'une cellule d'électrodialyse et un électrolyte est introduit dans le compartiment d'anode, le mélange de sel et d'électrolyte qui en résulte est alors soumis à l'électrolyse et l'acide téréphtalique résultant de la réaction des anions d'acide téréphtalique avec les cations de l'électrolyte dans le compartiment d'anode est retiré du compartiment d'anode et séparé de l'électrolyte par filtration.

2. Le procédé de récupération d'acide téréphtalique à partir de sa solution de sels en accord avec la revendication 1, **caractérisé par** le choix du sel d'acide téréphtalique parmi les sels d'ammonium, de sodium ou de potassium.

3. Le procédé de récupération d'acide téréphtalique à partir de sa solution de sels en accord avec la revendication 1 et 2, **caractérisé en ce que** l'électrolyte est choisi dans le groupe comprenant l'eau, un acide minéral ou un sel de celui-ci.

4. Le procédé de récupération d'acide téréphtalique à partir de sa solution de sels en accord avec quelconque des revendications 1 à 3, **caractérisé en ce que** la concentration de la solution aqueuse de l'acide téréphtalique soit comprise entre 2 et 15% en poids.

5. Le procédé de récupération d'acide téréphtalique à partir de sa solution de sels en accord avec quelconque des revendications 1 à 4, **caractérisé en ce que** l'électrolyte soit de l'acide sulfurique ou de l'acide nitrique d'une concentration de 0,01 M à 0,5 M.

6. Le procédé de récupération d'acide téréphtalique à partir de sa solution de sels en accord avec quelconque des revendications 1, 2 ou 4, **caractérisé en ce que** la solution aqueuse d'acide téréphtalique contienne en plus un sel d'un acide minéral à une concentration de 0,01 à 0,5% en poids.

7. Le procédé de récupération d'acide téréphtalique à partir de sa solution de sels en accord avec quelconque des revendications 1 à 6, **caractérisé en ce que** l'acide téréphtalique est retiré du compartiment de l'anode lorsque la courante électrodialyse descende à une valeur comprise entre 15 et 20% de sa valeur initiale.

8. Le procédé de récupération d'acide téréphtalique à partir de sa solution de sels en accord avec la revendication 1, **caractérisé en ce que** dans le processus d'hydroglycolyse le rapport pondéral du glycol à du téréphtalate de polyéthylène est de 1: 1 à 1: 10, de préférence de 1: 4 à 1: 6.

9. Le procédé de récupération d'acide téréphtalique à partir de sa solution de sels en accord avec la revendication 1 and 8, **caractérisé en ce que** la solution aqueuse contenant de l'hydroxyde et du glycol résultant du procédé de l'électrolyse est renvoyée à l'étape initiale d'hydroglycolyse.
